# EUROPÄISCHE PATENTSCHRIFT

(11) **EP 3 181 547 B1**
(45) Veröffentlichungstag und Bekanntmachung des Hinweises auf die Patenterteilung: **04.04.2018**
(21) Anmeldenummer: 15200511.2
(22) Anmeldetag: 16.12.2015
(51) Int. Cl.: C07C 67/38, C07C 69/42, C07C 69/34, C07C 69/44, C07C 69/612

(54) **VERFAHREN ZUR DOPPELTEN CARBONYLIERUNG VON ALLYLETHERN ZU ENTSPRECHENDEN DIESTERN**
METHOD FOR DOUBLE CARBONYLATION OF ALLYL ETHERS TO CORRESPONDING DIESTERS
PROCEDE DE CARBONYLATION DOUBLE D'ETHERS ALLYLIQUES EN DI-ESTERS CORRESPONDANTS

(43) Veröffentlichungstag der Anmeldung: 21.06.2017
(73) Patentinhaber: Evonik Degussa GmbH, 45128 Essen (DE)
(72) Erfinder: LIU, Jie, Hunan, 412100 (CN); HAOQUAN, Li, Xiaolan Town, 528415 Zhongshan (CN); BELLER, Matthias, 18211 Ostseebad Nienhagen (DE); JACKSTELL, Ralf, 27478 Cuxhaven Altenwalde (DE); FRANKE, Robert, 45772 Marl (DE); DYBALLA, Katrin Marie, 45657 Recklinghausen (DE)

(56) Entgegenhaltungen:
- JOHN F. KNIFTON: "Syngas reactions", JOURNAL OF ORGANOMETALLIC CHEMISTRY., Bd. 188, Nr. 2, 1. April 1980 (1980-04-01) , Seiten 223-236, XP55272822, CH ISSN: 0022-328X, DOI: 10.1016/S0022-328X(00)82815-6
- QIANG LIU ET AL: "Domino Catalysis: Palladium-Catalyzed Carbonylation of Allylic Alcohols to [beta],[gamma]-Unsaturated Esters", ANGEWANDTE CHEMIE INTERNATIONAL EDITION, Bd. 52, Nr. 31, 29. Juli 2013 (2013-07-29) , Seiten 8064-8068, XP055273131, DE ISSN: 1433-7851, DOI: 10.1002/anie.201303850

## Beschreibung

Die Erfindung betrifft ein Verfahren zur doppelten Carbonylierung von Allylethern zu den entsprechenden Diestern, wobei ein linearer oder verzweigter Allylether mit einem linearen oder verzweigtem Alkanol (Alkohol) unter Zuführung von CO und in Gegenwart eines katalytischen Systems aus einem Palladiumkomplex und mindestens einem organischen Phosphorliganden sowie in Anwesenheit eines Halogenwasserstoffs ausgewählt aus HCl, HBr oder HJ zur Reaktion gebracht wird.

Allylether stellen eine wichtige Klasse von organischen Zwischenprodukten in der Synthese von sowohl Großchemikalien als auch Feinchemikalien dar. Sie werden für die Synthese von pharmazeutischen Zwischenprodukten, Lösungsmittel, Farbstoffe, und funktionellen Materialien verwendet. Unter diesen Anwendungen ist die carbonylierende Umwandlung von Allylethern zu industriellen Estern einer der am häufigsten und praktischsten Syntheseroute, insbesondere wegen der enormen Nachfrage nach diesen Produkten in der Polymerisation [Werle, P.; Morawietz, M., "Alcohols, Polyhydric" in Ullmann's Encyclopedia of Industrial Chemistry: 2002, Wiley-VCH: Weinheim. 2002].
Die üblichen Methoden zu Diestern ausgehend von Allylethern benötigen zwei Schritte (Schema 1). Der erste Schritt, die Alkoxycarbonylierung zu β,γ ungesättigten Estern, wurde zuerst von Tsuji et al. 1964 beschrieben [Tsuji, J.; Kiji, J.; Imamura, S.; Morikawa, M., Organic Syntheses by Means of Noble Metal Compounds. VIII.1 Catalytic Carbonylation of Allylic Compounds with Palladium Chloride. Journal ofthe American Chemical Society 1964, 86 (20), 4350-4353]. In seinen Arbeiten zeigten verschiedene Allylverbindungen einschließlich Allylether gute Reaktivität und sind zu den entsprechenden β,γ ungesättigten Estern in Gegenwart von Palladiumchlorid als Katalysator umgewandelt worden. 1986 entwickelten Hanes et al. eine Syntheseroute für die Alkoxycarbonylierung von Allylethern unter Verwendung weniger teurer Katalysatoren, so z.B. Nickelhalogenide, Cobalthalogenide und Eisenhalogenide [Hanes, R. M.; Baugh, W. D., Carbonylation of allylic ethers to esters. US 4622416 A: 1986]. Später patentierten sie auch eine Methode zur Darstellung von Estern aus Allylethern in Gegenwart von Gruppe VIII Übergangsmetallen als Katalysator in Gegenwart von Halogeniden [Hanes, R. M.; Kwiatek, J., Carbonylation of allylic ethers to esters. US 5004568 A: 1991]. Beller et al. untersuchten vor kurzem den Mechanismus der Alkoxycarbonylierung von Allylalkoholen und Allenen, und zeigten, dass die gebildeten Allylether Intermediate in diesen Reaktionen darstellen [(a) Liu, J.; Liu, Q.; Franke, R.; Jackstell, R.; Beller, M., Ligand-Controlled Palladium-Catalyzed Alkoxycarbonylation of Allenes: Regioselective Synthesis of α,β- and β,γ-Unsaturated Esters (Journal ofthe American Chemical Society 2015, 137 (26), 8556-8563; (b) Liu, Q.; Wu, L.; Jiao, H.; Fang, X.; Jackstell, R.; Beller, M., Domino Catalysis: Palladium-Catalyzed Carbonylation of Allylic Alcohols to β,γ-Unsaturated Esters. Angewandte Chemie International Edition 2013, 52 (31), 8064-8068]. Der Zweite Schritt ist einfach die Alkoxycarbonylierung von β,γ-ungesättigten Estern zu Diestern (Schema 1). Dieser Schritt ist prinzipiell die Alkoxycarbonylierung von Alkenen, welche in einer Reihe von Publikationen und Patenten beschrieben werden [Reppe, W.; Kröper, H., Carbonylierung II. Carbonsäuren und ihre Derivate aus olefinischen Verbindungen und Kohlenoxyd. Justus Liebigs Annalen der Chemie 1953, 582 (1), 38-71]. 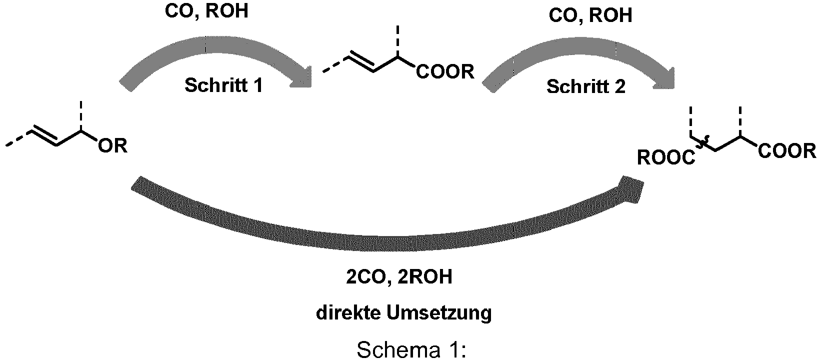 Knifton beschreibt die Carbonylierung von allylischen Verbindungen, J. Organomet. Chem. 1980, 188, 223-236.

Obwohl all die oben genannten Methoden gut entwickelt sind, und eine gute Reaktivität zur Synthese von Diestern aus Allylethern darstellen, erfordern sie zwei Reaktionsschritte, was einer breiten Anwendung in industriellen Anwendungen entgegensteht.

Der Erfindung lag deshalb die Aufgabe zugrunde nach effektiven Verfahren zur Synthese von Diestern zu suchen, die Zwischenschritte vermeidet.

Überraschend gelingt die Synthese von Diestern als eine Einschrittsynthese durch eine doppelte Carbonylierung von Allylethern. Die Aufgabe wird durch ein Verfahren gemäß Anspruch 1 gelöst. Die Unteransprüche stellen bevorzugte Verfahrensvarianten dar. Die erfindungsgemäß hergestellten Produkte - die Diester - liegen vorzugsweise als Isomerengemische vor.

Das Verfahren zur doppelten Carbonylierung von Allylethern zu Diestern ist dadurch gekennzeichnet, dass ein linearer oder verzweigter Allylether mit einem linearen oder verzweigtem Alkanol (Alkohol) unter Zuführung von CO und in Gegenwart eines katalytischen Systems aus einem Palladiumkomplex und mindestens einem organischen Phosphorliganden sowie in Anwesenheit eines Halogenwasserstoffs ausgewählt aus HCl, HBr oder HJ zur Reaktion gebracht wird. Bevorzugte Halogenwasserstoffe sind HCl und HBr.

Die verwendeten Allylether sind vorzugsweise Verbindungen der allgemeinen Formel (1) 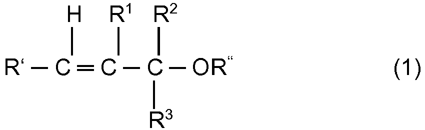
wobei R¹, R² und R³ unabhängig voneinander Wasserstoff oder einen C₁ bis C₁₀ Alkylrest bedeuten und
R' für Wasserstoff, oder einen gesättigten oder ungesättigten verzweigten oder unverzweigten aliphatischen, cycloaliphatischen oder cycloaliphatisch-aliphatischen Kohlenwasserstoffrest mit bis zu 12 C-Atomen, in dem C-C-Bindungen durch Sauerstoff oder die -O-CO-Gruppe unterbrochen sein können, oder einen Phenylrest steht, wobei der Phenylrest wie folgt substituiert sein kann: C₁- bis C₁₀-Alkyl- oder C₁- bis C₁₀-Alkoxy-Gruppen,
R" für einen gesättigten oder ungesättigten, verzweigten oder unverzweigten aliphatischen, cycloaliphatischen, araliphatischen, oder cycloaliphatisch-aliphatischen Kohlenwasserstoffrest mit bis zu 12 C-Atomen, in dem C-C-Bindungen durch Sauerstoff oder die -O-CO-Gruppe unterbrochen sein können.
R" vorzugsweise für einen C₁ bis C₁₂ Alkyl- oder Alkenylrest, einen C₄ bis C₂₀-Cycloalkylrest, oder eine C₇- bis C₁₁-Aralkylgruppe steht.

Alkyl bedeutet bevorzugt einen verzweigten oder unverzweigten Rest mit 1 bis 6 Kohlenstoffatomen. Alkylgruppen sind z.B. Methyl, Ethyl, Propyl, Isopropyl, 1-Butyl, 2- Butyl, 1-Pentyl, 1-Hexyl.

Die erfindungsgemäß eingesetzten Alkohole können primäre oder sekundäre Alkohole sein. Es können sowohl aliphatische, cycloaliphatische, aromatische als auch araliphatische Alkohole benutzt werden, vorzugsweise finden aliphatische, cycloaliphatiche und araliphatische Alkohole Anwendung. Im Allgemeinen werden Alkohole ROH im erfindungsgemäßen Verfahren verwendet, in denen der Rest R eine C₁- bis C₁₀-Alkyl-, eine C₄- bis C₂₀-Cycloalkyl- oder eine C₇- bis C₁₁-Aralkylgruppe ist.

Vorzugsweise werden Allylether der Formel (1) mit entsprechenden Alkoholen ROH umgesetzt, in denen R" dem Rest R entspricht.

Phenyl für R' und R in ROH können gegebenenfalls mit Substituenten wie C₁- bis C₁₀-AlkoxyGruppen substituiert sein.

Es werden vorzugsweise Alkohole ROH mit unsubstituierten Resten R verwendet. Selbstverständlich können auch Alkohole mit einer höheren Anzahl an Kohlenstoffatomen eingesetzt werden. Es werden vor allem niedere Alkanole (C₁ bis C₆) bevorzugt verwendet.

Beispiele für aliphatische Alkohole sind z. B. Methanol, Ethanol, 1-Propanol, 2-Propanol, C₄-Alkohole, z. B. 1-Butanol, 2-Butanol oder Isobutylalkohol, C₅-Alkohole, z. B. 1-Pentanol, Isoamylalkohol oder 2-Pentanol, C₆-Alkohole, z. B. 1-Hexanol, 2-Methyl-1-pentanol, 3-Methyl-1-pentanol, 2,2-Dimethyl-1-butanol, 2-Ethyl-1-butanol, 4-Ethyl-1-pentanol, 2-Hexanol, 3-Hexanol, 3-Methyl-2-pentanol, 2,3-Dimethyl-2-butanol, 2-Methyl-3-pentanol, 3-Methyl-3-pentanol, 4-Methyl-2-pentanol, 2-Methyl-2-pentanol, C₇-Alkohole, z. B. n-Heptylalkohol, 2-Methyl-1-hexylalkohol, 3-Methyl-1-hexylalkohol, 4-Methyl-1-hexylalkohol, 5-Methyl-1-hexylalkohol, 2-Ethyl-1-pentanol, 3-Ethyl-1-pentanol, 2,2-Di-methyl-1-pentanol, 3,3-Dimethyl-1-pentanol, 4,4-Dimethyl-1-pentanol, 2,3-Dimethyl-1-pentanol, 2,4-Dimethyl-1-pentanol, 3,4-Dimethyl-1-pentanol, C₈-Alkohole, z. B. 1-Octanol, 2-Methyl-1-heptanol, 3-Methyl-1-heptanol, 4-Methyl-1-heptanol, 5-Methyl-1-heptanol, 2-Octanol, 3-Octanol, 4-Octanol, 2-Methyl-2-heptanol, 3-Methyl-2-heptanol, 4-Methyl-2-heptanol, 5-Methyl-2-heptanol, 6-Methyl-2-heptanol, 2-Methyl-3-heptanol oder 3-Methyl-3-heptanol, und C₉-Alkohole, z. B. 1-Nonanol. Beispiele für die alicyclischen Alkohole mit 4 oder mehr Kohlenstoffatomen umfassen alicyclische Alkohole mit 4 bis 12 Kohlenstoffatomen, wie z. B. Cyclopentanol, Cyclohexanol oder Cyclooctanol.
Als C₇- bis C₁₁-Aralkylgruppe wird vorzugsweise die Benzylgruppe eingesetzt.

In einer Variante des Verfahrens wird die Reaktion in flüssiger Phase bei einer Temperatur von 70 bis 250 °C, vorzugsweise bei 80 bis 180 °C, besonders bevorzugt bei Temperaturen von 100 bis 150 °C durchgeführt.

Die Reaktion findet bevorzugt unter einem Druck von 2 bis 100 bar statt. Vorzugsweise wird die Reaktion bei einem Druck von 5 bis 50 bar durchgeführt. In einer Verfahrensvariante kann neben CO zusätzlich Stickstoff (N₂) aufgepresst werden, bevorzugt bei einem Druck p CO 40 bar + p N₂ 30 bar.

In einer Variante des Verfahren wird der Palladiumkomplex in-situ ausgehend von einem Vorkomplex gebildet wird, wobei als Palladiumquelle Palladium-enthaltende Salze und Komplexe als Vorstufe verwendet werden. Die Palladiumverbindungen können in unterschiedlichen Oxidationsstufen vorliegen, dabei sind vorteilhafterweise die Stufen 0 bis +II umfasst. Vorzugsweise ist der Palladiumkatalysator ausgewählt aus der Gruppe enthaltend Pd-Acetate, z.B. Pd(OAc)₂ und Pd(TFA)₂, Pd-Acetonate, z.B. Pd(acac)₂ und Pd₂(dba)₃, Pd-Halogenide und Pd-Halogenidkomplexe, z.B. PdCl₂, Pd(MeCN)₂Cl₂, [PdCl(C₃H₅)]₂ und Pd-Halogen-1,5-cyclooctadiene, wie Pd(cod)₂Cl₂, Pd-Nitrate, Pd-Oxid.

Die bevorzugten Phosphinliganden L weisen eine mono- oder bidentate Struktur auf. So werden z.B. die folgenden Liganden besonders vorteilhaft im erfindungsgemäßen Verfahren eingesetzt:
L1 - (9,9-Dimethyl-9H-xanthen-4,5-diyl)bis(diphenylphosphan) (= Xantphos),
L2 - (Oxybis(2,1-phenylen))bis(di-tert-butylphosphan) (= DPEphos),
L3 - 1,2-Bis((di-tert-butylphosphanyl)methyl)benzen,
L4 - Triphenylphosphin (= TPPO,
L5 - Di(1-adamantyl)-n-butyl-phosphin (= BuPAd₂).
Xantphos wird dabei besonders bevorzugt als Ligand eingesetzt.

Der Palladiumkatalysator umfasst den Phosphinliganden bevorzugt im Verhältnis Palladium zu Ligand im Bereich von 1:1 bis 1:20, vorzugsweise im Bereich von 1:1 bis 1:10, besonders bevorzugt im Bereich von 1:1 bis 1:3. Das Verhältnis von Palladium zu Halogenwasserstoff liegt bevorzugt im Bereich von 1:1 bis 1:20. Alle Verhältnisse sind Molverhältnisse.

Wirksame Katalysatormengen im Verfahren liegen vorzugsweise bei 0,01 bis 12 Mol-% Palladium bezogen auf den Alkohol, vorzugsweise werden 0,05 mol-% bis 1,5 mol-% Palladium, bezogen auf Alkohol eingesetzt.

Für das erfindungsgemäße Verfahren können Lösungsmittel eingesetzt werden. So werden beispielsweise polare inerte organische Lösungsmittel oder/und Wasser verwendet. Zum Beispiel werden dipolar aprotische Lösungsmittel, Ether, aliphatische Ether, Amide, aromatische Verbindungen, Alkohole und Ester, sowie deren Gemische verwendet. Besonders bevorzugt werden aromatische Verbindungen und aliphatische Ether wie Toluol und Diethylether eingesetzt.

Besonders bevorzugt wird im erfindungsgemäßen Verfahren als Halogenwasserstoff Chlorwasserstoff verwendet, vorzugsweise in einem apolaren organischen Lösungsmittel oder Lösungsmittelgemisch. Insbesondere wird die Reaktion in einem Gemisch aus HCl/Diethylether und einem weiteren Lösungsmittel, vorzugsweise Toluol, durchgeführt.

Überraschenderweise können mit dem erfindungsgemäßen Verfahren die entsprechenden Diester, in der Regel als Isomerengemische, aber auch als reine n-Verbindungen in guten Ausbeuten hergestellt werden. Das Verfahren stellt somit eine hoch atom- und prozessökonomische Diestersynthese dar. Überraschend werden Ausbeuten an Diestern bis zu 95% erreicht.

Die Erfindung wird in nachfolgenden Beispielen näher erläutert.

### Ausführungsbeispiele

### Allgemeine Bemerkungen:

Alle kommerziellen Reagenzien wurden von Alfa Aesar, Aldrich, TCI oder Strem bestellt. Soweit nicht anders gesagt, wurden handelsübliche Reagenzien ohne Reinigung verwendet. Der Allylether wird im Vakuum vor der Verwendung destilliert. Toluol, DMF, THF, Acetonitril und Methanol werden aus dem Lösungsmittelreinigungssystem PS-MD-7-von "Innovative technology" unter Anwendung von Standard-Schlenk-Techniken verwendet. Analytische Daten der Literatur-bekannten Verbindungen waren in Übereinstimmung mit gemeldeten Daten. NMR-Spektren wurden auf Bruker Avance 300 (300 MHz) NMR-Spektrometer aufgezeichnet. Multipletts wurden als s (Singulett), d (Dublett), t (Triplett), dd (Dublett von Dubletts), m (Multiplett) und br s (breites Singulett) zugeordnet. Alle Messungen wurden bei Raumtemperatur durchgeführt, sofern nicht anders angegeben. Elektronenstoß (EI) Massenspektren wurden auf AMD 402-Massenspektrometer (70 eV) aufgenommen. Hochauflösende Massenspektren (HRMS) wurden auf Agilent 6210 aufgezeichnet Time-of-Flight LC / MS (Agilent) mit Elektrospray-Ionisation (ESI). Die Daten werden als Masseeinheiten pro Ladung (m / z) und Intensitäten von Signalen in Klammern angegeben. Die Produkte wurden aus dem Reaktionsgemisch durch Säµlenchromatographie an Kieselgel 60, 0,063-0,2 mm, 70-230 mesh (Merck) getrennt.

### GC Analytik:

GC Analytik wurde mittels einem Agilent GC 7890A Gaschromatographen der Agilent Company mit einer 30 m HP-5 Säµle((Polydimethylsiloxan mit 5% Phenyl-Gruppen, 30 m, 0,32 mm ID, 0,25 & µm Filmdicke). durchgeführt. Temperaturprogramm: 35°C, 10 min; 10°C/min zu 285°C, 5 min; Injektionsvolumen 1 µL mit einem Split von 50:1.

### Abkürzungsverzeichnis:

BnOH: Benzylalkohol
CyOH: Cyclohexanol
equiv.: Äquivalente
HCl: Chlorwasserstoff
LSM: Lösungsmittel
THF: Tetrahydrofuran
T: Temperatur
p: Druck
Xantphos: 4,5-Bis(diphenylphosphanyl)-9,9-dimethylxanthen

### Beispiel 1

Umsetzung von Allylbutylether mit Butanol unter Verwendung von Pd(OAc)₂ und verschiedener Phosphin-Liganden sowie Chlorwasserstoff (Tabelle 1)

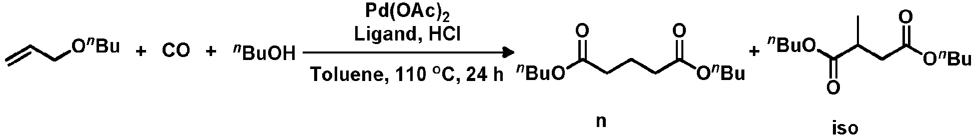

| Eintrag | Pd (mol%) | Ligand (mol%) | Lösungsmittel | Säure (mol%) | T (°C) | p(bar) | Ausbeute(***n-*/*iso*-**) |
|---|---|---|---|---|---|---|---|
| 1 | Pd(OAc)₂(1.0) | 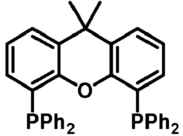 | Toluol | HCl (2.0) | 110 | 40 bar CO | 90% (48:52) |
| | | **L1** Xantphos (1.5 mol%) | | | | | |
| 2 | Pd(OAc)₂(1.0) | 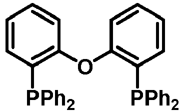 | Toluol | HCl (2.0) | 110 | 40 bar CO | 2% (70:30) |
| | | **L2** DPEphos (1.5 mol%) | | | | | |
| 3 | Pd(OAc)₂(1.0) | 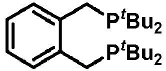 | Toluol | HCl (2.0) | 110 | 40 bar CO | 50% (36:64) |
| | | **L3** (1.5 mol%) | | | | | |
| 4 | Pd(OAc)₂(1.0) | 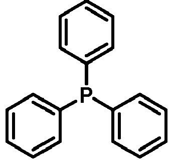 | Toluol | HCl (2.0) | 110 | 40 bar CO | 4% (22:78) |
| | | **L4** (3.0 mol%) | | | | | |
| 5 | Pd(OAc)₂(1.0) | 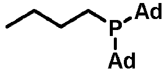 | Toluol | HCl (2.0) | 110 | 40 bar CO | 4% (23:77) |
| | | **L5** (3.0 mol%) | | | | | |

### Beispiel 1.1

### Tabelle 1, Eintrag 1

Ein 4 ml Glasvial wird mit [Pd(acac)₂] (2.24mg, 1 mol%), L1 (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (137 µl, 1.5 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nach dem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 1.2

### Tabelle 1, Eintrag 2

Ein 4 ml Glasvial wird mit [Pd(acac)₂] (2.24mg, 1 mol%), L2 (8.1 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (137 µl, 1.5 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 1.3

### Tabelle 1, Eintrag 3

Ein 4 ml Glasvial wird mit [Pd(acac)₂] (2.24mg, 1 mol%), L3 (5,9 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (137 µl, 1.5 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 1.4

### Tabelle 1, Eintrag 4

Ein 4 ml Glasvial wird mit [Pd(acac)₂] (2.24mg, 1 mol%), L4 (7.9 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (137 µl, 1.5 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 1.5

### Tabelle 1, Eintrag 5

Ein 4 ml Glasvial wird mit [Pd(acac)₂] (2.24mg, 1 mol%), L5 (10.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (137 µl, 1.5 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 2

### Variation der Säuremenge (Tabelle 2)

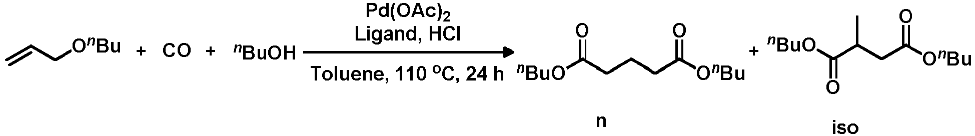

**Tabelle 2**

| Eintrag | Pd (mol%) | Ligand (mol%) | Lösungsmittel | Säure(mol%) | T (°C) | p (bar) | Ausbeute (***n-*/*iso-***) |
|---|---|---|---|---|---|---|---|
| 1 | Pd(acac)₂(1.0) | Xantphos(1.5) | Toluol | HCl (1.0) | 110 | 40 | 25% (51:49) |
| 2 | Pd(acac)₂(1.0) | Xantphos(1.5) | Toluol | HCl (1.5) | 110 | 40 | 63% (49:51) |
| 3 | Pd(acac)₂(1.0) | Xantphos(1.5) | Toluol | HCl (2.0) | 110 | 40 | 78% (53:47) |
| 4 | Pd(acac)₂(1.0) | Xantphos(1.5) | Toluol | HCl (2.5) | 110 | 40 | 67% (48:52) |
| 5 | Pd(acac)₂(1.0) | Xantphos(1.5) | Toluol | HCl (3.0) | 110 | 40 | 22% (48:52) |
| 6 | Pd(acac)₂(1.0) | Xantphos(1.5) | Toluol | HCl (4.0) | 110 | 40 | 8% (46:54) |

### Beispiel 2.1

### Tabelle 2, Eintrag 1

Ein 4 ml Glasvial wird mit [Pd(acac)₂] (3.07mg, 1 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (10 µl, 1 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 2.2

### Tabelle 2, Eintrag 2

Ein 4 ml Glasvial wird mit [Pd(acac)₂] (3.07mg, 1 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (15 µl, 1.5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 2.3

### Tabelle 2, Eintrag 3

Ein 4 ml Glasvial wird mit [Pd(acac)₂] (3.07mg, 1 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 2.4

### Tabelle 2, Eintrag 4

Ein 4 ml Glasvial wird mit [Pd(acac)₂] (3.07mg, 1 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (25µl, 2.5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 2.5

### Tabelle 2, Eintrag 5

Ein 4 ml Glasvial wird mit [Pd(acac)₂] (3.07mg, 1 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (30 µl, 3 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 2.6

### Tabelle 2, Eintrag 6

Ein 4 ml Glasvial wird mit [Pd(acac)₂] (3.07mg, 1 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (40 µl, 4 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 3

### Variation der Säuren (Tabelle 3)

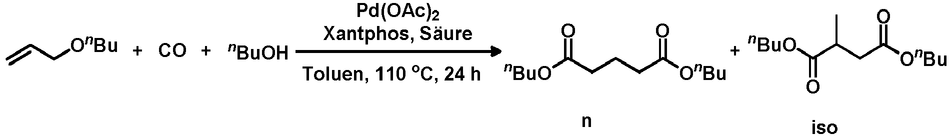

**Tabelle 3**

| Eintra g | Pd (mol%) | Ligand (mol%) | LSM | Säure (mol%) | T (°C) | p (bar) | Ausbeute (***n-*/*iso-***) |
|---|---|---|---|---|---|---|---|
| 1 | Pd(OAc)₂(1.0) | Xantphos(1.5) | Toluol | HCl (2.0) | 110 | 40 | 90% (47:53) |
| 2 | Pd(OAc)₂(1.0) | Xantphos(1.5) | Toluol | H₂SO₄ (1.0) | 110 | 40 | 0 (-) |
| 3 | Pd(OAc)₂(1.0) | Xantphos(1.5) | Toluol | HOAc (2.0) | 110 | 40 | 0 (-) |
| 4 | Pd(OAc)₂(1.0) | Xantphos(1.5) | Toluol | CF₃COO H (2.0) | 110 | 40 | 0 (-) |
| 5 | Pd(OAc)₂(1.0) | Xantphos(1.5) | Toluol | CH₃SO₃H (2.0) | 110 | 40 | 0 (-) |
| 6 | Pd(OAc)₂(1.0) | Xantphos(1.5) | Toluol | CF₃SO₃H (2.0) | 110 | 40 | 0 (-) |
| 7 | Pd(OAc)₂(1.0) | Xantphos(1.5) | Toluol | PTSA·H₂ O (2.0) | 110 | 40 | 0 (-) |
| 8 | Pd(OAc)₂(1.0) | Xantphos(1.5) | Toluol | - | 110 | 40 | 0 (-) |
| 9 | Pd(OAc)₂(1.0) | Xantphos(1.5) | Aceton | HCl (aq) (2.0) | 110 | 40 | 55 |
| 10 | Pd(OAc)₂(1.0) | Xantphos(1.5) | Aceton | HBr (aq) (2.0) | 110 | 40 | 11 |

### Beispiel 3.1

### Tabelle 3, Eintrag 1

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 3.2

### Tabelle 3, Eintrag 2

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 0.5 M H₂SO₄ Diethyletherlösung(20 µl, 1.0 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 3.3

### Tabelle 3, Eintrag 3

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und HOAc (1.1 µl, 2.0 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 3.4

### Tabelle 3, Eintrag 4

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und CF₃COOH (1.6 µl, 2.0 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 3.5

### Tabelle 3, Eintrag 5

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und CH₃SO₃H (1.3 µl, 2.0 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 3.6

### Tabelle 3, Eintrag 6

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und CF₃SO₃H (1.8 µl, 2.0 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 3.7

### Tabelle 3, Eintrag 7

Ein 4 ml Glasvial wird mit [Pd(OAc)₂] (2.24 mg, 1.0 mol%), Xantphos (L1) (8.7 mg, 1.5 mol%), PTSA·H₂O (3.8 mg, 2.0 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 3.8

### Tabelle 3, Eintrag 8

Ein 4 ml Glasvial wird mit [Pd(OAc)₂] (2.24 mg, 1.0 mol%), Xantphos (L1) (8.7 mg, 1.5 mol%), und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 3.9

### Tabelle 3, Eintrag 9

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Aceton, n-Butanol (137 µl, 1.5 mmol), Allylbutylether (145 µl, 1 mmol), und 1 M HCL (wässrige Lösung) (20 µl, 2.0 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 3.10

### Tabelle 3, Eintrag 10

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Aceton, n-Butanol (137 µl, 1.5 mmol), Allylbutylether (145 µl, 1 mmol), und 1 M HBr (wässrige Lösung) (20 µl, 2.0 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 4

### Variation der Palladiummenge und des Butanols (Tabelle 4)

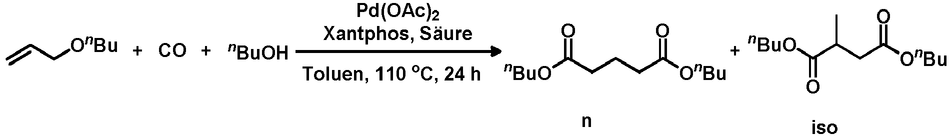

**Tabelle 4**

| Eintrag | Pd (mol%) | Ligand (mol%) | *n*Butanol (mol% ) | Lösung s-mittel | Säure (mol%) | T(°C ) | P (bar) | Ausbeute (***n-*/*iso-***) |
|---|---|---|---|---|---|---|---|---|
| 1 | Pd(acac)₂(1.0) | Xantphos(1.5) | 120 | Toluol | HCl (2.0) | 110 | 40 | 79% (45:55) |
| 2 | Pd(acac)₂(1.0) | Xantphos(1.5) | 150 | Toluol | HCl (2.0) | 110 | 40 | 91% (46:54) |
| 3 | Pd(acac)₂(1.0) | Xantphos(1.5) | 200 | Toluol | HCl (2.0) | 110 | 40 | 85% (48:52) |
| 4 | Pd(acac)₂(0.5) | Xantphos(0.75) | 200 | Toluol | HCl (1.0) | 110 | 40 | 33% (50:50) |
| 5 | Pd(acac)₂(0.25 ) | Xantphos(0.375 ) | 200 | Toluol | HCl (0.5) | 110 | 40 | 6% (51:49) |

### Beispiel 4.1

### Tabelle 4, Eintrag 1

Ein 4 ml Glasvial wird mit [Pd(acac)₂] (3.07mg, 1 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (109 µl, 1.2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt

### Beispiel 4.2

### Tabelle 4, Eintrag 2

Ein 4 ml Glasvial wird mit [Pd(acac)₂] (3.07mg, 1 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (137 µl, 1.5 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 4.3

### Tabelle 4, Eintrag 3

Ein 4 ml Glasvial wird mit [Pd(acac)₂] (3.07mg, 1 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 4.4

### Tabelle 4, Eintrag 4

Ein 4 ml Glasvial wird mit Xantphos (L1) (4,35 mg, 0.75 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt.. [Pd(acac)₂] (500 µl, 0.01M in Toluol, 1.53 mg, 0.5 mol%), Toluol (1.5 mL), Allylbutylether (145 µl, 1.0 mmol), n-Butanol (182 µl, 2.0 mmol) Und 1 M HCl-Diethyletherlösung (10 µl, 1.0 mol%) werden mittels einer Spritze injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 4.5

### Tabelle 4, Eintrag 5

Ein 4 ml Glasvial wird mit Xantphos (L1) (2.18 mg, 0.375 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. [Pd(acac)₂] (250 µl, 0.01M in Toluol, 0.765mg, 0.25 mol%), Toluol (1.5 mL), Allylbutylether (145 µl, 1.0 mmol), n-Butanol (182 µl, 2.0 mmol) und 1 M HCl Diethyletherlösung (5 µl, 1.0 mol%) werden mittels einer Spritze injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 5

Variation der Xantphosmenge (Tabelle 5)

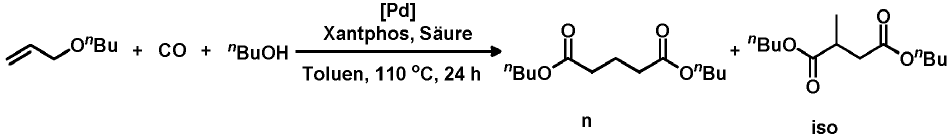

**Tabelle 5**

| Eintrag | Pd (mol%) | Ligand (mol%) | LSM | Säure (mol%) | T(°C) | p (bar) | Ausbeute (***n-*/*iso-***) |
|---|---|---|---|---|---|---|---|
| 1 | Pd(acac)₂(1.0) | Xantphos(1.0) | Toluol | HCl (2.0) | 110 | 40 | 7% (48:52) |
| 2 | Pd(acac)₂(1.0) | Xantphos(1.5) | Toluol | HCl (2.0) | 110 | 40 | 85% (47:53) |
| 3 | Pd(acac)₂(1.0) | Xantphos(2.0) | Toluol | HCl (2.0) | 110 | 40 | 62% (49:51) |

### Beispiel 5.1

### Tabelle 5, Eintrag 1

Ein 4 ml Glasvial wird mit [Pd(acac)₂] (3.07mg, 1 mol%), Xantphos (L1) (5.8 mg, 1.0 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 5.2

### Tabelle 5, Eintrag 2

Ein 4 ml Glasvial wird mit [Pd(acac)₂] (3.07mg, 1 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 5.3

### Tabelle 5, Eintrag 3

Ein 4 ml Glasvial wird mit [Pd(acac)₂] (3.07mg, 1 mol%), Xantphos (L1) (11.6 mg, 2 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 6

### Variation des Palladiumprecursors (Tabelle 6)

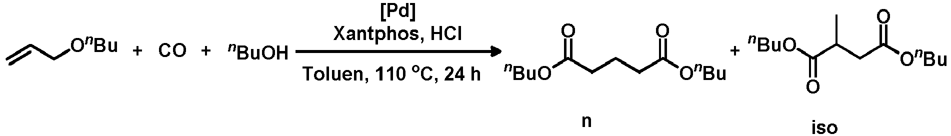

**Tabelle 6**

| Eintrag | Pd (mol%) | Ligand (mol%) | *n*Butanol (mol%) | LSM | Säure( mol%) | T(°C) | p (bar) | Ausbeute(***n* -/*iso*-**) |
|---|---|---|---|---|---|---|---|---|
| 1 | Pd(acac)₂(1.0) | Xantphos(1.5) | 200 | Toluol | HCl (2.0) | 110 | 40 | 85% (53:47) |
| 2 | Pd(OAc)₂(1.0) | Xantphos(1.5) | 200 | Toluol | HCl (2.0) | 110 | 40 | 89% (48:52) |
| 3 | PdCl₂(1.0) | Xantphos(1.5) | 200 | Toluol | HCl (2.0) | 110 | 40 | 90% (45:55) |
| 4 | Pd(MeCN)₂Cl₂ (1.0) | Xantphos(1.5) | 200 | Toluol | HCl (2.0) | 110 | 40 | 90% (44:56) |
| 5 | Pd(cod)₂Cl₂(1. 0) | Xantphos(1.5) | 200 | Toluol | HCl (2.0) | 110 | 40 | 85% (45:55) |
| 6 | [PdCl(C₃H₅)]₂( 1.0) | Xantphos(1.5) | 200 | Toluol | HCl (2.0) | 110 | 40 | 87% (48:52) |
| 7 | Pd₂(dba)₃(1.0) | Xantphos(1.5) | 200 | Toluol | HCl (2.0) | 110 | 40 | 82% (49:51) |
| 8 | Pd(TFA)₂ | Xantphos(1.5) | 200 | Toluol | HCl (2.0) | 110 | 40 | 93% (49:51) |
| 9 | Pd(acac)₂(1.0) | Xantphos(1.5) | 150 | Toluol | HCl (2.0) | 110 | 40 bar CO+30 bar N2 | 87% (45:55) |
| 10 | Pd(OAc)₂(1.0) | Xantphos(1.5) | 150 | Toluol | HCl (2.0) | 110 | 40 bar CO+30 bar N2 | 92% (45:55) |
| 11 | Pd(TFA)₂(1.0) | Xantphos(1.5) | 150 | Toluol | HCl (2.0) | 110 | 40 bar CO+30 bar N2 | 94% (47:53) |
| 12 | PdO (1.0) | Xantphos(1.5) | 200 | Toluol | HCl (2.0) | 110 | 40 bar CO | 73% (48:52) |
| 13 | Pd(NO₃)₂· 2H₂O (1.0) | Xantphos(1.5) | 200 | Toluol | HCl (2.0) | 110 | 40 bar CO | 74% (49:51) |

### Beispiel 6.1

### Tabelle 6, Eintrag 1

Ein 4 ml Glasvial wird mit [Pd(acac)₂] (3.07mg, 1 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 6.2

### Tabelle 6, Eintrag 2

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 6.3

### Tabelle 6, Eintrag 3

Ein 4 ml Glasvial wird mit [PdCl₂] (1.76 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt

### Beispiel 6.4

### Tabelle 6, Eintrag 4

Ein 4 ml Glasvial wird mit [Pd(MeCN)₂Cl₂] (2.58 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 6.5

### Tabelle 6, Eintrag 5

Ein 4 ml Glasvial wird mit [Pd(cod)₂Cl₂] (2.85 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 6.6

### Tabelle 6, Eintrag 6

Ein 4 ml Glasvial wird mit [PdCl(C₃H₅)]₂ (1.83 mg, 0.5 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 6.7

### Tabelle 6, Eintrag 7

Ein 4 ml Glasvial wird mit [Pd₂(dba)₃] (4.58 mg, 0.5 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 6.8

### Tabelle 6, Eintrag 8

Ein 4 ml Glasvial wird mit [Pd(TFA)₂] (3.32 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 6.9

### Tabelle 6, Eintrag 9

Ein 4 ml Glasvial wird mit [Pd(acac)₂] (3.07 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (136 µl, 1,5 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO und 30 bar Stickstoff (Reinheit 5.0)aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 6.10

### Tabelle 6, Eintrag 10

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (136 µl, 1,5 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO und 30 bar Stickstoff (Reinheit 5.0) aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 6.11

### Tabelle 6, Eintrag 11

Ein 4 ml Glasvial wird mit [Pd(TFA)₂] (3.32 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (136 µl, 1,5 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO und 30 bar Stickstoff (Reinheit 5.0) aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 6.12

### Tabelle 6, Eintrag 12

Ein 4 ml Glasvial wird mit [PdO] (1.2 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 6.13

### Tabelle 6, Eintrag 13

Ein 4 ml Glasvial wird mit [Pd(NO₃)₂·2H₂O] (2.65 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 7

### Variation des Lösungsmittels, Tabelle 7

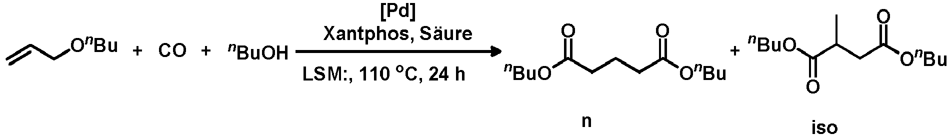

**Tabelle 7**

| Eintrag | Pd (mol%) | Ligand (mol%) | LSM | Säure (mol%) | Temp (°C) | pbar) | Ausbeute (***n-*/*iso-***) |
|---|---|---|---|---|---|---|---|
| 1 | Pd(acac)₂(1.0) | Xantphos(1.5) | Toluol | HCl (2.0) | 110 | 40 | 85% (47:53) |
| 2 | Pd(acac)₂(1.0) | Xantphos(1.5) | THF | HCl (2.0) | 110 | 40 | 27% (45:55) |
| 3 | Pd(acac)₂(1.0) | Xantphos(1.5) | MeCN | HCl (2.0) | 110 | 40 | 6% (66:34) |
| 4 | Pd(acac)₂(1.0) | Xantphos(1.5) | Heptan | HCl (2.0) | 110 | 40 | 18% (57:43) |
| 5 | Pd(acac)₂(1.0) | Xantphos(1.5) | Aceton | HCl (2.0) | 110 | 40 | 60% (54:46) |
| 6 | Pd(acac)₂(1.0) | Xantphos(1.5) | Dioxan | HCl (2.0) | 110 | 40 | 20% (48:52) |
| 7 | Pd(acac)₂(1.0) | Xantphos(1.5) | *n*-Butanol | HCl (2.0) | 110 | 40 | 6% (76:24) |

### Beispiel 7.1

### Tabelle 7, Eintrag 1

Ein 4 ml Glasvial wird mit [Pd(acac)₂] (3.07 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 7.2

### Tabelle 7, Eintrag 2

Ein 4 ml Glasvial wird mit [Pd(acac)₂] (3.07 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml THF, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 7.3

### Tabelle 7, Eintrag 3

Ein 4 ml Glasvial wird mit [Pd(acac)₂] (3.07 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml MeCN, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 7.4

### Tabelle 7, Eintrag 4

Ein 4 ml Glasvial wird mit [Pd(acac)₂] (3.07 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Heptan, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt

### Beispiel 7.5

### Tabelle 7, Eintrag 5

Ein 4 ml Glasvial wird mit [Pd(acac)₂] (3.07 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Aceton, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 7.6

### Tabelle 7, Eintrag 6

Ein 4 ml Glasvial wird mit [Pd(acac)₂] (3.07 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Dioxan, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt

### Beispiel 7.7

### Tabelle 7, Eintrag 7

Ein 4 ml Glasvial wird mit [Pd(acac)₂] (3.07 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml n-Butanol, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 8

### Substratvariation (Tabelle 8)

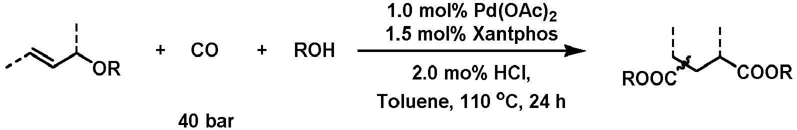

**Tabelle 8**

| Eintrag | Substrat | Alkohol | Hauptprodukte | Ausbeute(**n-/*iso-***) |
|---|---|---|---|---|
| 1 | 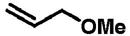 | MeOH (1.5 equiv.) | 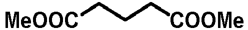 | 81% (50:50) |
| | | | 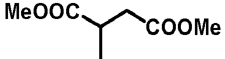 | |
| 2 | 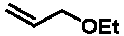 | EtOH (1.5 equiv.) | 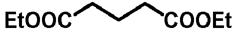 | 83% (46:54) |
| | | | 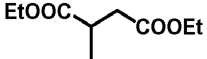 | |
| 3 | 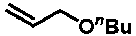 | *ⁿ*BuOH (1.5 equiv.) | 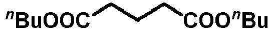 | 90% (48:52) |
| | | | 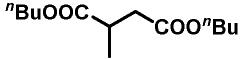 | |
| 4 | 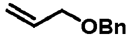 | BnOH (1.5 equiv.) | 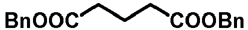 | 70% (70%*^{b}*) (48:52) |
| | | | 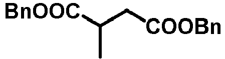 | |
| 5 | 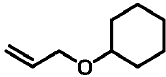 | 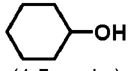 | 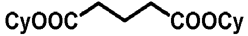 | 61% (53:47) |
| | | (1.5 equiv.) | 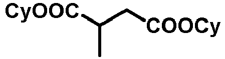 | |
| 6 | 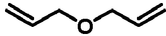 | *ⁿ*BuOH (6.0 equiv.) | 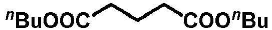 | 93% (54:46) |
| | | | 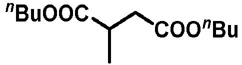 | |
| 7 | 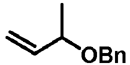 | BnOH (1.5 equiv.) | 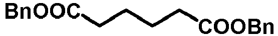 | 61% (6:94) |
| | | | 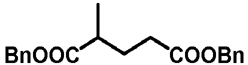 | |
| | | | 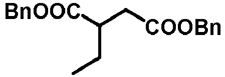 | |
| 8 | 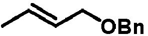 | BnOH (1.5 equiv.) | 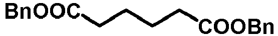 | 44% (7:93) |
| | | | 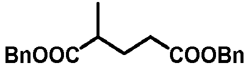 | |
| | | | 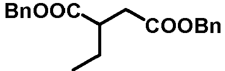 | |
| 9*^{a}* | 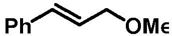 | MeOH (1.5 equiv.) | 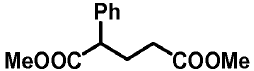 | 35%*^{b}* (69:31) |
| | | | 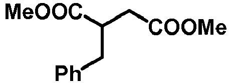 | |

| | | | | |
|---|---|---|---|---|
| *^{a}* 4.0 mol% HCl, 48 h *^{b}* Isolierte Ausbeute | | | | |

### Beispiel 8.1

### Tabelle 8, Eintrag 1

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, Methylallylether (72 mg, 1.0 mmol), Methanol (48 mg, 1.5 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 8.2

### Tabelle 8, Eintrag 2

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, Ethylallyether (86 mg, 1.0 mmol), Ethanol (69 mg, 1.5 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 8.3

### Tabelle 8, Eintrag 3

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butylallylether (114 mg, 1.0 mmol), n-Butanol (111mg, 1.5 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 8.4

### Tabelle 8, Eintrag 4

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, Benzylallylether (148 mg, 1.0 mmol), BnOH (162 mg, 1.5 mmol) und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 8.5

### Tabelle 8, Eintrag 5

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, Cyclohexylallylether(140 mg, 1.0 mmol), CyOH (150 mg, 1.5 mmol) und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 8.6

### Tabelle 8, Eintrag 6

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, Diallylether (98 mg, 1.0 mmol), n-Butanol (444 mg, 6.0 mmol) und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 8.7

### Tabelle 8, Eintrag 7

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, But-3-en-2-yloxy)methyl)benzen (162 mg, 1.0 mmol), BnOH (162 mg, 1.5 mmol) und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 8.8

### Tabelle 8, Eintrag 8

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, (But-2-en-1-yloxy)methyl)benzen (162 mg, 1.0 mmol), BnOH (162 mg, 1.5 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 8.9

### Tabelle 8 Eintrag 9

Ein 4 ml Glasvial wird mit [Pd(OAc)₂] (2.24 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, (3-Methoxyprop-1-en-1-yl)benzen (148 mg, 1.0 mmol), MeOH (48 mg, 1.5 mmol) und 2 M HCl-Diethyletherlösung (20 µl, 4.0 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 9

### Temperaturvariation (Tabelle 9)

| Eintrag | Pd (mol%) | Ligand (mol%) | LSM | Säure (mol%) | Temp (°C) | p(bar) | Ausbeute (***n-*/*iso-***) |
|---|---|---|---|---|---|---|---|
| | | | | | | | |
| 1 | Pd(acac)₂(1.0) | Xantphos(1.5) | Toluole | HCl (2.0) | 100 | 40 | 64% (48:52) |
| 2 | Pd(acac)₂(1.0) | Xantphos(1.5) | Toluole | HCl (2.0) | 110 | 40 | 85% (53:47) |
| 3 | Pd(acac)₂(1.0) | Xantphos(1.5) | Toluole | HCl (2.0) | 120 | 40 | 77% (48:52) |
| 4 | Pd(acac)₂(1.0) | Xantphos(1.5) | Toluole | HCl (2.0) | 140 | 40 | 75% (48:52) |

### Beispiel 9.1

### Tabelle 9, Eintrag 1

Ein 4 ml Glasvial wird mit [Pd(acac)₂] (3.07 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Propylencarbonat, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 100°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 9.2

### Tabelle 9, Eintrag 2

Ein 4 ml Glasvial wird mit [Pd(acac)₂] (3.07 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Propylencarbonat, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 110°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 9.3

### Tabelle 9, Eintrag 3

Ein 4 ml Glasvial wird mit [Pd(acac)₂] (3.07 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Propylencarbonat, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 120°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

### Beispiel 9.4

### Tabelle 9, Eintrag 4

Ein 4 ml Glasvial wird mit [Pd(acac)₂] (3.07 mg, 1.0 mol%), Xantphos (L1) (8.7 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Propylencarbonat, n-Butanol (182 µl, 2 mmol), Allylbutylether (145 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 140°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Dodecan (100 µl) als interner Standard zugesetzt. Die Ausbeute und Selektivität wird mittels GC Analyse bestimmt.

## Patentansprüche

1. Verfahren zur doppelten Carbonylierung von Allylethern zu Diestern,
**dadurch gekennzeichnet, dass** ein linearer oder verzweigter Allylalkohol mit einem linearen oder verzweigtem Alkanol unter Zuführung von CO und in Gegenwart eines katalytischen Systems aus einem Palladiumkomplex und mindestens einem organischen Phosphorliganden sowie in Anwesenheit eines Halogenwasserstoffs ausgewählt aus HCl, HBr oder HJ zur Reaktion gebracht wird.

2. Verfahren nach Anspruch 1,
**dadurch gekennzeichnet, dass** die Allylether Verbindungen der allgemeinen Formel (1) darstellen: 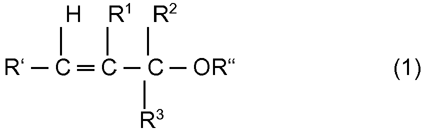
wobei R¹, R² und R³ unabhängig voneinander Wasserstoff oder einen C₁ bis C₁₀ Alkylrest bedeuten und
R' für Wasserstoff, oder einen gesättigten oder ungesättigten verzweigten oder unverzweigten aliphatischen, cycloaliphatischen oder cycloaliphatisch-aliphatischen Kohlenwasserstoffrest mit bis zu 12 C-Atomen, in dem C-C-Bindungen durch Sauerstoff oder die -O-CO-Gruppe unterbrochen sein können, oder einen Phenylrest steht, wobei der Phenylrest wie folgt substituiert sein kann: C₁- bis C₁₀-Alkyl- oder C₁-bis C₁₀-Alkoxy-Gruppen,
R" für einen gesättigten oder ungesättigten, verzweigten oder unverzweigten aliphatischen, cycloaliphatischen, araliphatischen, oder cycloaliphatisch-aliphatischen Kohlenwasserstoffrest mit bis zu 12 C-Atomen, in dem C-C-Bindungen durch Sauerstoff oder die -O-CO-Gruppe unterbrochen sein können.

3. Verfahren nach Anspruch 1 oder 2,
**dadurch gekennzeichnet, dass** die Alkanole Verbindungen der allgemeinen Formel ROH darstellen, wobei
R eine C₁- bis C₁₀-Alkyl-, eine C₄- bis C₂₀-Cycloalkyl- oder eine C₇- bis C₁₁-Aralkylgruppe ist.

4. Verfahren nach einem der Ansprüche 1 bis 3,
**dadurch gekennzeichnet, dass** die Reaktion in flüssiger Phase bei einer Temperatur von 70 bis 250 °C durchgeführt wird.

5. Verfahren nach einem der Ansprüche 1 bis 4,
**dadurch gekennzeichnet, dass** Reaktion unter einem Druck von 2 bis 100 bar durchgeführt wird.

6. Verfahren nach einem der Ansprüche 1 bis 5,
**dadurch gekennzeichnet, dass** der Palladiumkomplex in-situ ausgehend von einem Vorkomplex gebildet wird, wobei als Palladiumquelle Palladium-enthaltende Salze und Komplexe als Vorstufe verwendet werden.

7. Verfahren nach Anspruch 6,
**dadurch gekennzeichnet, dass** der Palladiumkomplex ausgewählt ist aus der Gruppe enthaltend Pd-Acetate, Pd-Acetonate, Pd-Halogenide und Pd-Halogenidkomplexe, sowie Pd-Halogen-1,5-cyclooctadiene, Pd-Nitrate und Pd-Oxid.

8. Verfahren nach einem der Ansprüche 1 bis 7,
**dadurch gekennzeichnet, dass** die Phosphinliganden eine mono- oder bidentate Struktur aufweisen, vorzugsweise ausgewählt sind aus der Gruppe umfassend
L1 - (9,9-Dimethyl-9H-xanthen-4,5-diyl)bis(diphenylphosphan),
L2 - (Oxybis(2,1-phenylen))bis(di-tert-butylphosphan),
L3 - 1,2-Bis((di-tert-butylphosphanyl)methyl)benzen,
L4 - Triphenylphosphin,
L5 - Di(1-adamantyl)-n-butyl-phosphin.

9. Verfahren nach einem der Ansprüche 1 bis 8,
**dadurch gekennzeichnet, dass** als Halogenwasserstoff Chlorwasserstoff verwendet wird.

10. Verfahren nach einem der Ansprüche 1 bis 9,
**dadurch gekennzeichnet, dass** das Verhältnis von Palladium zu Halogenwasserstoff im Bereich von 1: 1 bis 1:20 liegt.

## Claims

1. Process for doubly carbonylating allyl ethers to diesters,
**characterized in that** a linear or branched allyl alcohol is reacted with a linear or branched alkanol with supply of CO and in the presence of a catalytic system composed of a palladium complex and at least one organic phosphorus ligand and in the presence of a hydrogen halide selected from HCl, HBr and HI.

2. Process according to Claim 1,
**characterized in that** the allyl ethers are compounds of the general formula (1): 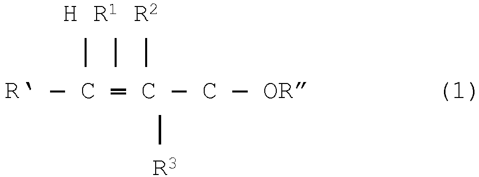
where R¹, R² and R³ are independently hydrogen or a C₁ to C₁₀ alkyl radical and
R' is hydrogen, or a saturated or unsaturated, branched or unbranched, aliphatic, cycloaliphatic or cycloaliphatic-aliphatic hydrocarbyl radical having up to 12 carbon atoms, in which C-C bonds may be interrupted by oxygen or the -O-CO- group, or a phenyl radical, where the phenyl radical may be substituted as follows: C₁ - to C₁₀-alkyl or C₁ - to C₁₀-alkoxy groups,
R" is a saturated or unsaturated, branched or unbranched, aliphatic, cycloaliphatic, araliphatic or cycloaliphatic-aliphatic hydrocarbyl radical having up to 12 carbon atoms, in which C-C bonds may be interrupted by oxygen or the -O-CO- group.

3. Process according to Claim 1 or 2,
**characterized in that** the alkanols are compounds of the general formula ROH where
R is a C₁- to C₁₀-alkyl, a C₄- to C₂₀-cycloalkyl or a C₇- to C₁₁-aralkyl group.

4. Process according to any of Claims 1 to 3,
**characterized in that** the reaction is conducted in the liquid phase at a temperature of 70 to 250°C.

5. Process according to any of Claims 1 to 4,
**characterized in that** reaction is conducted under a pressure of 2 to 100 bar.

6. Process according to any of Claims 1 to 5,
**characterized in that** the palladium complex is formed in situ proceeding from a pre-complex, using, as palladium source, palladium-containing salts and complexes as precursor.

7. Process according to Claim 6,
**characterized in that** the palladium complex is selected from the group comprising Pd acetates, Pd acetonates, Pd halides and Pd halide complexes, and also Pd-halogen-1,5-cyclooctadienes, Pd nitrates and Pd oxide.

8. Process according to any of Claims 1 to 7,
**characterized in that** the phosphine ligands have a mono- or bidentate structure, preferably selected from the group comprising
L1 - (9,9-dimethyl-9H-xanthene-4,5-diyl)bis(diphenylphosphine),
L2 - (oxybis(2,1-phenylene))bis(di-tert-butylphosphine),
L3 - 1,2-bis((di-tert-butylphosphinyl)methyl)benzene,
L4 - triphenylphosphine,
L5 - di(1-adamantyl)-n-butylphosphine.

9. Process according to any of Claims 1 to 8, **characterized in that** the hydrogen halide used is hydrogen chloride.

10. Process according to any of Claims 1 to 9, **characterized in that** the ratio of palladium to hydrogen halide is in the range from 1:1 to 1:20.

## Revendications

1. Procédé de carbonylation double d'éthers allyliques en diesters, **caractérisé en ce qu'**un alcool allylique linéaire ou ramifié est mis en réaction avec un alcanol linéaire ou ramifié avec introduction de CO et en présence d'un système catalytique constitué par un complexe de palladium et au moins un ligand phosphore organique, et en présence d'un halogénure d'hydrogène choisi parmi HCl, HBr ou HI.

2. Procédé selon la revendication 1, **caractérisé en ce que** les éthers allyliques sont des composés de formule générale (1) : 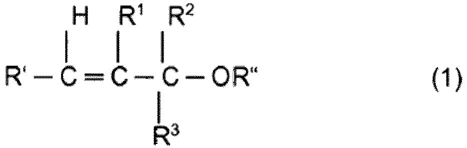
dans laquelle R¹, R² et R³ signifient indépendamment les uns des autres l'hydrogène ou un radical alkyle en C₁ à C₁₀, et
R' représente l'hydrogène ou un radical hydrocarboné saturé ou insaturé, ramifié ou non ramifié, aliphatique, cycloaliphatique ou cycloaliphatique-aliphatique, contenant jusqu'à 12 atomes C, dans lequel les liaisons C-C peuvent être interrompues par de l'oxygène ou le groupe -O-CO-, ou un radical phényle, le radical phényle pouvant être substitué de la manière suivante : groupes alkyle en C₁ à C₁₀ ou alcoxy en C₁ à C₁₀,
R" représente un radical hydrocarboné saturé ou insaturé, ramifié ou non ramifié, aliphatique, cycloaliphatique, araliphatique ou cycloaliphatique-aliphatique, contenant jusqu'à 12 atomes C, dans lequel les liaisons C-C peuvent être interrompues par de l'oxygène ou le groupe -O-CO-.

3. Procédé selon la revendication 1 ou 2, **caractérisé en ce que** les alcanols sont des composés de formule générale ROH, dans laquelle
R est un groupe alkyle en C₁ à C₁₀, cycloalkyle en C₄ à C₂₀ ou aralkyle en C₇ à C₁₁.

4. Procédé selon l'une quelconque des revendications 1 à 3, **caractérisé en ce que** la réaction est réalisée en phase liquide à une température de 70 à 250 °C.

5. Procédé selon l'une quelconque des revendications 1 à 4, **caractérisé en ce que** la réaction est réalisée sous une pression de 2 à 100 bar.

6. Procédé selon l'une quelconque des revendications 1 à 5, **caractérisé en ce que** le complexe de palladium est formé in situ à partir d'un pré-complexe, des sels et des complexes contenant du palladium étant utilisés en tant que source de palladium en tant que précurseurs.

7. Procédé selon la revendication 6, **caractérisé en ce que** le complexe de palladium est choisi dans le groupe contenant les acétates de Pd, les acétonates de Pd, les halogénures de Pd et les complexes d'halogénures de Pd, ainsi que les halogéno-1,5-cyclooctadiènes de Pd, les nitrates de Pd et l'oxyde de Pd.

8. Procédé selon l'une quelconque des revendications 1 à 7, **caractérisé en ce que** les ligands phosphine présentent une structure mono- ou bidentate, sont de préférence choisis dans le groupe comprenant
L1 - le (9,9-diméthyl-9H-xanthène-4,5-diyl)bis(diphénylphosphane),
L2 - 1'(oxybis(2,1-phénylène))bis(di-tert-butylphosphane),
L3 - le 1,2-bis((di-tert-butylphosphanyl)méthyl)benzène,
L4 - la triphénylphosphine,
L5 - la di(1-adamantyl)-n-butyl-phosphine.

9. Procédé selon l'une quelconque des revendications 1 à 8, **caractérisé en ce que** du chlorure d'hydrogène est utilisé en tant qu'halogénure d'hydrogène.

10. Procédé selon l'une quelconque des revendications 1 à 9, **caractérisé en ce que** le rapport entre le palladium et l'halogénure d'hydrogène se situe dans la plage allant de 1:1 à 1:20.
